# EUROPEAN PATENT APPLICATION

(11) **EP 2 258 324 A1**
(43) Date of publication of application: **08.12.2010**
(21) Application number: 09162019.5
(22) Date of filing: 05.06.2009
(51) Int. Cl.: A61F 5/01, A61F 5/042

(54) **Orthosis for a human leg**

(71) Applicant: Rijksuniversiteit Groningen, 9712 CP Groningen (NL); ACADEMISCH ZIEKENHUIS GRONINGEN, 9713 GZ Groningen (NL)
(72) Inventor: Verkerke, Gijsbertus Jacobus, 9756 TG, Glimmen (NL); Kuijer, Roel, 9713 TN, Groningen (NL); Van der Werf, Maarten Wobbe, 7514 DK, Enschede (NL); Langius, Rolf Willem, 6811 HC, Arnhem (NL); Hekman, Edsko Evert Geert, 7534 CH, Enschede (NL); Rietman, Johan Swanik, 7531 JM, Enschede (NL); Bulstra, Sjoerd, 9713 HM, Groningen (NL)
(74) Representative: Hatzmann, Martin

(57) **Abstract**

An orthosis (1) for a leg of a human body comprises an above knee body support (2) for supporting a portion of said human body above the leg's knee; a below foot ground support (3) for resting upon the ground (8) while not supporting the leg's foot (9); a connection mechanism (4) interconnecting said body support and said ground support and being suitable for transferring loads via the ground support to the ground, while bypassing the knee and the foot, said mechanism comprising a pair of strut assemblies (11, 21), each strut assembly comprising a hinge (14, 24) arranged to be pivotable proximate the knee to permit bending of the knee during walking; and at least one distraction element (5, 6) for exerting a force on the below the knee portion of the leg for pulling apart opposing condyles of at least one condylar joint in the knee.

## Description

The invention relates to an orthosis for a leg of a human body. More in particular, the invention relates to such an orthosis for counteracting osteoarthritis in the knee.

Damage of joint tissues usually starts with damage to articular cartilage. The ultimate result, degenerative joint disease or osteoarthritis, is frequently occurring. Approximately 80% of people over 60 years of age have osteoarthritis features in X-rays of their knees and hip joints. The ultimate treatment is inevitably a total joint prosthesis.

Osteoarthritis in the knee can occur on three different joints within the knee: medial tibial-femoral, lateral tibial-femoral and the patello-femoral joint.

An orthosis for a leg of a human body is for example known from US 6,010,474.

It is an object of the invention to provide an improved solution for counteracting osteoarthritis in the knee.

For that purpose the invention provides an orthosis according to claim 1.

At least in an operative walking mode of such an orthosis according to the invention, the below foot ground support can rest upon the ground while not supporting the leg's foot. The connection mechanism then transfers loads from the supported body portion above the knee via the below foot ground support to the ground, while the knee and the foot are bypassed in such manner that no such load is transferred via the knee and via the foot. Thus, the knee is totally spared from such loads.

In fact, because of the at least one distraction element, the opposing condyles of at least one condylar joint in the knee are, at least locally in the knee, being pulled apart.

Since each strut assembly is arranged to be pivotable proximate the knee to permit bending of the knee during walking, motion of the knee is preserved.

Thus, the orthosis according to the invention offers an improved treatment of osteoarthritis. The treatment offers unloading and distraction of the knee joint, while preserving motion of the knee. The unloading and distraction prevent further damage to the cartilage and create favourable conditions for restoration, even under muscle activity. Motion of the knee joint enables transportation of nutrition to the damaged area, as well as removal of waste products. Normally it may be expected that, because of the synergistic effects of said unloading, said distraction and said preservation of knee motion, restoration of the cartilage can be obtained in 2-3 months.

When applying the orthosis known from US 6,010,474, the foot is still supported by a shoe and heel bridge assembly and parts of the loads from the supported body portion above the knee are in fact transferred via the knee and the foot. As a consequence, the use of this known orthosis still results in compressive forces between opposing condyles of at least one condylar joint in the knee. In contrast to this known orthosis, the use of the orthosis according to the invention totally eliminates such compressive forces and in fact even results in oppositely directed distraction forces. These distinguishing features and effects of the orthosis according to the invention, in combination with the preservation of motion of the knee, are responsible for an improved restoration of the cartilage.

In a preferable embodiment the at least one distraction element is arranged for letting, at least in the operative walking mode, said exerted force act on the below the knee portion of the leg in such direction that free movements of the below the knee portion of the leg relative to the upper leg are at least partially restricted in a direction from the below foot ground support towards said hinges.

The nature of the distraction provided by this type of distraction element is such that it gives a general positive effect for all condylar joints in the knee, i.e. the medial tibial-femoral joint, the lateral tibial-femoral joint and the patello-femoral joint.

In a further preferable embodiment the at least one distraction element is arranged for letting, at least in the operative walking mode, said exerted force act on the below the knee portion of the leg in such direction that the range of motion of the knee is at least partially restricted in a pivotal direction towards full extension of the leg.

The nature of the distraction provided by this type of distraction element is such that it gives a positive effect for at least one of the condylar joints in the knee. In addition, this type of distraction element counteracts osteoarthritis in the knee because it unloads the knee in two additional respects. The first respect is unloading of the passive knee stabilizers. The second respect is unloading of the active knee stabilizers.

The passive knee stabilizers are the various ligaments, parts of tendons and the joint capsule. The tension from the passive stabilizers is the highest at full extension and full flexion. At a flexion of about 30 degrees the knee has its maximum laxity. With lower tension of the passive knee stabilizers there is less loading on the joint surfaces. Hence, by at least partially restricting the range of flexion of the knee in a pivotal direction towards full extension of the leg, part of the tensioning of the passive stabilizers is avoided.

With active knee stabilizers there is meant musculature attached to or nearby the knee with tendons. Muscles have two functions with respect to the knee, i.e. stabilizing and moving. Tendons which cross the joint will be tensioned by activation of the muscles and thereby give stability to the knee joint. By at least partially restricting the range of flexion of the knee in a pivotal direction towards full extension of the leg, the patient is less motivated to use his/her thigh musculature for stabilizing during the stance phase.

In a further preferable embodiment the at least one distraction element comprises resilient means for resiliently exerting said exerted force. Such resilient means enable some fluctuations in the pulled apart positions of the opposing condyles, as well as provide damping of such fluctuating movements.

In a further preferable embodiment the at least one distraction element is adjustable for adjusting said exerted force for adjusting said pulling apart. In this way it is for example possible to adjust said pulling apart in dependence of the progress of the restoration process. For example, the adjustment can be performed on a regular basis, for instance such that the condyles are being pulled a little further apart every day, once or twice a week, or with another frequency. Such adjustment on a regular basis promotes, in some cases, the speed of the restoration process.

Preferably, the orthosis further comprises at least one automatically driven actuator for realizing said adjusting of said at least one distraction element.

Preferably, the orthosis further comprises measuring means for measuring conditions of the leg or of the orthosis, such as conditions of the knee or conditions of the leg's musculature or conditions of the pivotal position of at least one of the strut assemblies, and further comprises control means for automatically controlling said actuator in dependence on said measured conditions. Preferably, said measured conditions comprise electrical muscle activity of the leg's musculature. With the use of such measuring means and such control means, it is for example possible to temporarily increase distraction in reaction to a temporary increase of knee loading due to changes in muscle activity or due to changes in the extent of flexion of the leg.

In a further preferable embodiment at least one of said hinges comprises automatic locking-and-unlocking means for locking and unlocking the pivotability of said hinge(s) based on said transferred load at least in such manner that, at least in the operative walking mode, unlocking occurs during at least part of a swing-through phase of a walking cycle of the leg and locking occurs during at least part of a phase of such walking cycle in which the below foot ground support is supported by the ground. Hinges with such automatic locking-and-unlocking means are available in the market. The application of such hinge(s) reduces muscle activity around the knee as the patient does not need to stabilize the knee with his/her muscles.

Further details, aspects and embodiments of the invention will be described, by way of example only, with reference to the schematic figures in the enclosed drawing.
Fig. 1 shows, in perspective view, an example of an embodiment of an orthosis according to the invention.
Fig. 2 shows, in perspective view, part of a below foot ground support of the orthosis of Fig. 1, wherein a distraction element is attached to the below foot ground support.

The shown orthosis 1 comprises an above knee body support 2 and a below foot ground support 3. The ground support 3 is suitable, at least in an operative walking mode of the orthosis 1, for resting upon the ground 8 while not supporting the leg's foot 9.

The orthosis 1 furthermore comprises a connection mechanism 4 interconnecting said body support 2 and said ground support 3. The mechanism 4 is suitable, at least in said mode, for transferring loads from said supported body portion above the knee via the ground support 3 to the ground 8, while bypassing the knee and the foot in such manner that no such load is transferred via the knee and via the foot.

The mechanism 4 comprises a pair of strut assemblies 11, 21 for extending along the leg on opposite lateral sides of the leg. Each strut assembly 11, 21 comprises a hinge 14, 24 arranged to be pivotable proximate the knee to permit bending of the knee during walking. In the shown example, the strut assembly 11 comprises an upper strut 12, a lower strut 15 and a hinge 14 pivotably interconnecting the upper strut 12 and the lower strut 15. Similarly, the strut assembly 21 comprises an upper strut 22, a lower strut 25 and a hinge 24 pivotably interconnecting the upper strut 22 and the lower strut 25. Alternatively, it is also possible that such hinges are directly connected to the body support 2 or to another type of such an above knee body support, in which case the upper struts 12, 22 of the strut assemblies can be omitted.

In the shown example, the body support 2 is based upon a custom made prosthetic socket as used by trans-femoral amputees. It is handmade by an orthopedic instrument maker. The prosthetic socket is a plastic molded on a plaster cast of the leg. The functioning of this solution for transferring ground forces to the ischial tuberosity is proven by the use of it by all trans-femoral prosthetics. This solution gives the best and most comfortable force transfer to the ischial tuberosity.

The commonly used prosthetic socket is redesigned to comply with several demands for the orthosis. With a common socket the residual limb slides in the socket. The socket is a shell with one opening at the top. This means that the socket for the orthosis has to have a second opening on the condylar side and it has to be put on in a different way. There are several design options for putting the socket on. First, the socket can be split longitudinally. In this way the socket can be pulled apart and wrapped around the upper leg. Another option is to make the opening on the condylar side larger creating a passage for a stretched foot. The patient can put this socket on like a trouser-leg. The advantage of the last option is the higher torsion stiffness by the intact shell. Another advantage is that the rest of the orthosis can stay mounted. With the first option dismantling of the orthosis is needed to create the possibility of pulling the socket apart. Dismantling is needed because the rest of the orthosis can not be pulled apart, since that would not create a stable orthosis.

The option which may be preferable is the intact shell which results in a stable construction and is easy to put on. One of the design problems of this option is the opening at the condylar side, it is partly in conflict with the demand of a condylar fit. An opening only on the end under the condyles will be too small. Therefore a cut-out to the posterior side is created. The shape of this cut-out is dependent on the diameter of the upper leg and the foot size. With the plaster cast the orthopedic instrument maker can measure the necessary dimensions and calculate the enlargement needed for the passage of the foot. The opening created will weaken the construction near the condyles. The construction can bulge out by the missing material on the posterior side. To prevent this, a strap can be placed over the gap between the two condyles. Another option to create the space and still have a support on the condyles is leaving a certain amount of space between the condyles and the shell. When the socket is in place, sacs can be filled with air/gel to fill the gap creating the support on the condyles. An additional feature of such air/gel sacs is the possibility to prevent sliding of the socket.

Instead of the shown example, however, various other types of above knee body supports can be applied in an orthosis according to the invention. For example, the specific types of thigh cuffs disclosed in the abovementioned document US 6,010,474 can be used. In principle, it is even possible to apply above knee body supports which are invasively attached to a portion of a human body above the leg's knee.

In the shown example, the hinges are not directly mounted on the socket. The hinges need to have some clearance between relative to the knee. The clearance can not be created when the hinges are directly connected to the socket.

Mounting of the orthosis on the hinges can be done by bars with a width of for example between 16 and 20 millimeters. These widths are normal for the bars used in orthoses. A logical solution for the connection is to use the same bars in this orthosis. Standard orthosis bars are made from stainless steel. Because of the weight bearing function of this orthosis the 20 millimeter may be preferable. This kind of bars is bendable in a workshop, making aligning to the socket easy.

The bars are attached to the hinges with bolts and nuts, delivered with the hinges. Mounting on the socket can be done in the same way. A different option is to attach the bars on the socket with rivets. Orthopedic instrument makers use this type of attachment in comparable products. An advantage of rivets is the low assembly height in comparison with nuts and bolts. The disadvantage of rivets, that they can not be released, does not apply because the bar does not need to be released. Preferably, the rivets or bolts which connect the bars to the socket must be spaced apart as far as possible.

Various types of hinges can be applied in an orthosis according to the invention. Also, various suitable types of hinges are available in the market.

Preferably, at least one of the hinges 14, 24 comprises automatic locking-and-unlocking means for locking and unlocking the pivotability of said hinge(s) based on said transferred load at least in such manner that, at least in an operative walking mode of the orthosis 1, unlocking occurs during at least part of a swing-through phase of a walking cycle of the leg and locking occurs during at least part of a phase of such walking cycle in which the below foot ground support is supported by the ground. Hinges with such automatic locking-and-unlocking means are available in the market and are favourable in view of the hinge's function to control flexion. The use of one or two such "controlling" hinge(s) reduces the forces of the thigh musculature. Normally, the thighbone muscles are activated during stance phase for stabilizing the walking movement, but with a lock this is not necessary anymore. This locking therefore relieves the knee of a part of the muscle load.

The lower struts 15, 25 are arranged such that there is created space between the foot 9 and the ground 8. To support the lower struts 15, 25 against torsion, two metal arches 26 and 27 are interconnectingly placed between the lower struts 15, 25.

The below foot ground support 3 has an interface to the lower struts 15, 25 (preferably with vertical length adjustment means 31), as well as an interface to the ground 8. Its design preferably is directed to creating a normal walking pattern.

Various types of below foot ground supports can be applied in an orthosis according to the invention, preferably for example in accordance with the following demands. Adequate stiffness of the ground support 3 is necessary for several reasons. The orthosis may not bend, because in this way the foot will come on the ground. Furthermore, it will contribute to the stiffness of the complete construction from the hinges down. For a transfer of the forces it must sustain the forces without breaking and with a very low deformation. Besides having enough strength and stiffness the construction also has to be lightweight. The next demand is related to the walking pattern. The distance between the foot and the ground makes the orthosis longer than the leg. A heightening is needed on the other leg to compensate that, making the distance directly related to the heightening. Since it is not comfortable to walk on shoes with a high heel beneath it, the distance must be kept as small as possible. The part 10 of the ground support 3 that touches the ground 8 preferably has a certain curvature in the walking direction. The curvature will create a smoother movement, resembling the normal walking pattern. The mounting of the ground support to the lower struts must be on a certain height to give clearance for the length adjusting of the lower part of the orthosis. At the mounting position the ground support is wider than on the ground. The reason for this width difference is that there has to be reckoned with a normal shoe width.

The orthosis 1 furthermore comprises at least one distraction element attached to the ground support 3 and/or to a portion of the connection mechanism 4 below said hinges 14, 24. The at least one distraction element is suitable, at least in said operative walking mode of the orthosis 1, for exerting a force on the below the knee portion of the leg for pulling apart, at least locally in the knee, opposing condyles of at least one condylar joint in the knee.

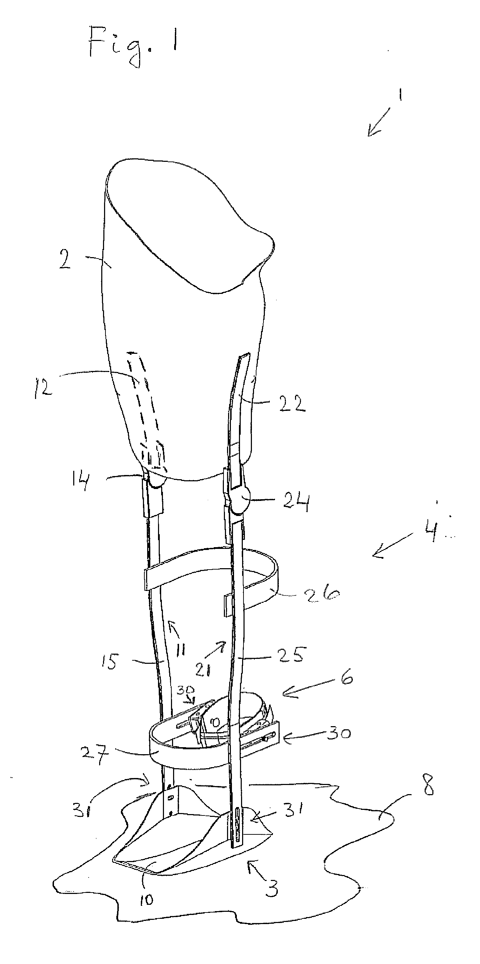

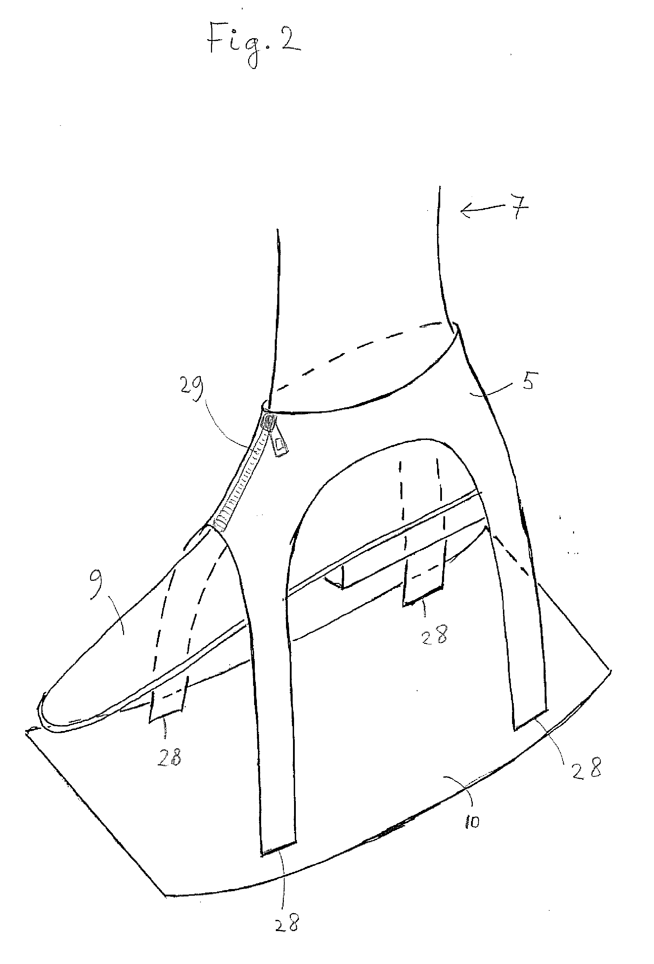

As mentioned, the at least one distraction element may be arranged for letting, at least in an operative walking mode of the orthosis 1, said exerted force act on the below the knee portion of the leg in such direction that free movements of the below the knee portion of the leg relative to the upper leg are at least partially restricted in a direction from the below foot ground support 3 towards said hinges 14, 24. Such a distraction element is shown in Fig. 2 by means of the reference numeral 5. For simplicity, the distraction element 5 has been omitted in Fig. 1, although it is supposed to be present there. The shown distraction element 5 is designed to hold the foot 9 of the leg 7 such that the mentioned force will be exerted on the below the knee portion of the leg. In the shown example, this is realized in that the shown distraction element 5 is a flexible structure and is attached, at a number of positions 28, to the upperside of part 10 of the ground support 3. As shown, the flexible structure has a zip 29.

As mentioned, the at least one distraction element may comprise resilient means for resiliently exerting said exerted force. In the case of the shown distraction element 5 this can for example be realized in that the flexible structure comprises elastic material or in that one or more spring element(s) are connected to the flexible structure.

As mentioned, the at least one distraction element may be arranged for letting, at least in an operative walking mode of the orthosis 1, said exerted force act on the below the knee portion of the leg in such direction that the range of flexion of the knee is at least partially restricted in a pivotal direction towards full extension of the leg. Such a distraction element is shown in Fig. 1 by means of the reference numeral 6. The shown distraction element 6 is designed to hold the lower leg of the leg 7 such that the mentioned force will be exerted on the below the knee portion of the leg. In the shown example, this is realized in that the shown distraction element 6 is a holder for the lower leg, which holder is attached to the arch 27 that interconnects the lower struts 15, 25. This holder 6 is arranged such that the longitudinal directions of the lower struts 15, 25 of the orthosis 1 each are at an inclined angle relative to the longitudinal direction of the lower leg of the patient, also during pivotal movements about the axes of the hinges 14, 24. This means that, when the lower struts 15, 25 are in a full extension position in the sense of the orthosis 1, the lower leg of the patient is not in its full extension position, but still has a certain degree of flexion. Preferably, the position of the holder 6 relative to the arch 27 is adjustable. For that purpose suitable adjustment means may be provided, indicated in Fig. 1 by reference numeral 30. Through these adjustment means 30 the said inclined angle between the longitudinal directions of the lower struts, on the one hand, and the lower leg of the patient, on the other hand, can be adjusted.

The adjustment means 30 are an example of an orthosis, wherein the at least one distraction element is adjustable for adjusting said exerted force for adjusting said pulling apart.

As mentioned, the orthosis may comprise at least one automatically driven actuator for realizing the adjusting of the at least one distraction element. Such an automatically driven actuator can for example make use of an electromotor.

When using such an actuator, the orthosis preferably further comprises measuring means for measuring conditions of the leg or of the orthosis, such as conditions of the knee or conditions of the leg's musculature or conditions of the pivotal position of at least one of the strut assemblies, and further comprises control means for automatically controlling said actuator in dependence on said measured conditions. Preferably, said measured conditions comprise electrical muscle activity of the leg's musculature (EMG-signal). With the use of such measuring means and such control means, it is for example possible to temporarily increase distraction in reaction to a temporary increase of knee loading due to changes in muscle activity or due to changes in the extent of flexion of the leg.

In the foregoing specification, the invention has been described with reference to specific examples of embodiments of the invention. It will, however, be evident that various modifications and changes may be made therein without departing from the broader scope of the invention as set forth in the appended claims.

For example, it is possible to apply various other types of distraction elements. For example, distraction elements of the type that restrict free movements of the below the knee portion of the leg in a direction from the below foot ground support towards the hinges may also be connected to the connection mechanism instead of to the ground support. Furthermore, distraction elements of the type that restrict the range of flexion of the knee in a pivotal direction towards full extension of the leg may also be connected to the ground support instead of to the connection mechanism. Furthermore, it is possible to combine these functions of both these two types of distraction elements in a single distraction element, which may be connected either to the ground support or to the connection mechanism, or to both the ground support and the connection mechanism.

The said adjustability of all possible distraction elements may be realized by means of adjustability of relative positions of (parts of) the distraction elements within the orthosis and/or by means of adjustability of spring forces of spring elements applied in such distraction elements.

However, other modifications, variations and alternatives are also possible. The specifications and drawings are, accordingly, to be regarded in an illustrative rather than in a restrictive sense.

## Claims

1. Orthosis for a leg (7) of a human body, comprising:
- an above knee body support (2) suitable, at least in an operative walking mode of the orthosis (1), for supporting a portion of said human body above the leg's knee;
- a below foot ground support (3) suitable, at least in said mode, for resting upon the ground (8) while not supporting the leg's foot (9);
- a connection mechanism (4) interconnecting said above knee body support and said below foot ground support, the mechanism being suitable, at least in said mode, for transferring loads from said supported body portion above the knee via the below foot ground support to the ground, while bypassing the knee and the foot in such manner that no such load is transferred via the knee and via the foot, said mechanism comprising a pair of strut assemblies (11, 21) for extending along the leg on opposite lateral sides of the leg, each strut assembly comprising a hinge (14, 24) arranged to be pivotable proximate the knee to permit bending of the knee during walking; and
- at least one distraction element (5, 6) attached to the below foot ground support and/or to a portion of the connection mechanism below said hinges, the at least one distraction element being suitable, at least in said mode, for exerting a force on the below the knee portion of the leg for pulling apart, at least locally in the knee, opposing condyles of at least one condylar joint in the knee.

2. Orthosis according to claim 1, wherein the at least one distraction element (5) is arranged for letting, at least in said mode, said exerted force act on the below the knee portion of the leg in such direction that free movements of the below the knee portion of the leg relative to the upper leg are at least partially restricted in a direction from the below foot ground support (3) towards said hinges (14, 24).

3. Orthosis according to claim 1 or 2, wherein the at least one distraction element (6) is arranged for letting, at least in said mode, said exerted force act on the below the knee portion of the leg in such direction that the range of motion of the knee is at least partially restricted in a pivotal direction towards full extension of the leg (7).

4. Orthosis according to any one of the preceding claims, wherein the at least one distraction element (5) comprises resilient means for resiliently exerting said exerted force.

5. Orthosis according to any one of the preceding claims, wherein the at least one distraction element (6) is adjustable for adjusting said exerted force for adjusting said pulling apart.

6. Orthosis according to claim 5, further comprising at least one automatically driven actuator for realizing said adjusting of said at least one distraction element (5, 6).

7. Orthosis according to claim 6, further comprising measuring means for measuring conditions of the leg (7) or of the orthosis (1), such as conditions of the knee or conditions of the leg's musculature or conditions of the pivotal position of at least one of the strut assemblies (11, 21), and further comprising control means for automatically controlling said actuator in dependence on said measured conditions.

8. Orthosis according to claim 7, wherein said measured conditions comprise electrical muscle activity of the leg's musculature.

9. Orthosis according to any one of the preceding claims, wherein at least one of said hinges (14, 24) comprises automatic locking-and-unlocking means for locking and unlocking the pivotability of said hinge(s) based on said transferred load at least in such manner that, at least in said mode, unlocking occurs during at least part of a swing-through phase of a walking cycle of the leg (7) and locking occurs during at least part of a phase of such walking cycle in which the below foot ground support (3) is supported by the ground (8).
